# EUROPÄISCHE PATENTSCHRIFT

(11) **EP 0 362 857 B1**
(45) Veröffentlichungstag und Bekanntmachung des Hinweises auf die Patenterteilung: **29.12.1993**
(21) Anmeldenummer: 89118526.6
(22) Anmeldetag: 05.10.1989
(51) Int. Cl.: C07C 11/04, C07C 7/11

(54) **Verfahren und Vorrichtung zur Aufarbeitung eines C2-Stromes**
Process and apparatus for processing a C2 stream
Procédé et appareillage pour le traitement d'un courant en C2

(30) Priorität: 05.10.1988 DE 3833795
(43) Veröffentlichungstag der Anmeldung: 11.04.1990
(73) Patentinhaber: Linde Aktiengesellschaft, 65189 Wiesbaden (DE)
(72) Erfinder: Haehn, Peter Clemens, Dipl.-Ing., D-8192 Geretsried (DE)
(74) Vertreter: Schaefer, Gerhard, Dr.

(56) Entgegenhaltungen:
- DE-A- 3 413 898
- DE-B- 2 410 713
- US-A- 3 098 107
- US-A- 3 837 144

## Beschreibung

Die Erfindung betrifft ein Verfahren sowie eine Vorrichtung zur Aufarbeitung eines C₂-Stromes, der neben Ethylen als Hauptkomponente noch 15 - 30 % Ethan, 1 - 3 % Acetylen und Spuren von Propan/Propylen enthält, mittels Rektifikation bei Drücken von 4 bis 35 bar, wobei eine Kopffraktion, bestehend aus Ethylen und Acetylen, sowie eine Sumpffraktion, bestehend aus Ethan und Propan/ Propylen, gewonnen wird, wobei die Kopffraktion anschließend zur Abtrennung des Acetylens einer Wäsche mit einem organischen Lösungsmittel unterworfen wird, welches nach Aufnahme des Acetylens sowie teilweise mitgelöstem Ethylens einer Regenerierung unter Freisetzung der gelösten Gase unterzogen und zur Wäsche rückgeführt wird und wobei das Kopfprodukt der Waschsäule nach Kühlung und teilweiser Kondensation zum Teil als Rücklauf in den oberen Teil der Waschsäule aufgegeben wird.

Acetylen als auch Ethylen sind wichtige Grundstoffe der industriellen organischen Chemie, weshalb eine möglichst reine Darstellung dieser Stoffe angestrebt wird.

Aus der eigenen US-A-3 098 107 ist ein Verfahren zur Ethylen-Gewinnung aus einem Wasserstoff, Stickstoff, Methan, Ethylen, Ethan, Propylen, Butan und in nur geringem Maße Acetylen enthaltendem Gasgemisch bekannt. In mehreren aufeinanderfolgenden Trenn- und Trockenschritten werden zunächst C₃₊-Anteile und anschließend Wasserstoff, Stickstoff und Methan abgetrennt. Aus dem anfallenden lediglich Ethylen, Ethan und Acetylen enthaltendem Gemisch wird durch Rektifikation bei einem Druck von etwa 1,3 bar Ethan abgetrennt. Acetylen wird anschließend in einer Wäsche mit Aceton bei einem Druck zwischen etwa 3 und 4 bar vom Ethylen ausgewaschen. Mit dem Acetylen ausgewaschenes Ethylen wird über einen Aufkocher in die Wäsche zurückgeführt.

Gasgemische, aus denen mit Hilfe von Tieftemperaturprozessen reines Ethylen gewonnen werden kann, werden heute durch thermische Spaltung von Erdöl- oder Erdgasfraktionen bei hohen Temperaturen unter Zusatz von Wasserdampf hergestellt. Beispielsweise führt die Spaltung einer Naphthafraktion zu einer erhöhten Ethylenbildung gegenüber C₃-/C₄-Olefinen. In dem derart erhaltenen C₂-Schnitt steigt der Gehalt an C₂H₂ bis auf 1 - 3 Gew.% bezogen auf C₂H₄ an.

Ein derart zusammengesetztes Gasgemisch wird üblicherweise anschließend in einem oder mehreren Wärmetauschern abgekühlt, so daß sich ein Teil - vorwiegend höherer Kohlenwasserstoffe - verflüssigt, der in einem nachfolgenden Abscheider abgetrennt wird.

Das verbliebene Gasgemisch wird stufenweise auf Drücke zwischen 4 und 35 bar verdichtet, wodurch sich die Kondensation der Komponenten von tiefen zu weniger tiefen Temperaturen verschiebt, und rektifikatorisch in eine Ethylen-Kopffraktion und eine Ethan-Sumpffraktion zerlegt.

Bisher ist es üblich, das Acetylen vor der Fraktionierung des C₂-Schnittes mit N-Methylpyrrolidon (NMP) oder Dimethylformamid (DMF) auszuwaschen, wobei der C₂-Schnitt etwa 68 bis 84% C₂H₄, 14 bis 30% C₂H₆ und 2 % C₂H₂ enthält. Acetylen löst sich im Lösungsmittel und kann mit 99,5% Reinheit gewonnen werden.

Diese Art der C₂H₂-Abtrennung ist beispielsweise in "Industrielle Organische Chemie", 3. Auflage 1988, Verlag Chemie, K. Weissermel, H. J. Arpe, Seiten 104 - 105, beschrieben.

Nun hat dieses bekannte Verfahren den großen Nachteil, daß sich im C₂-Schnitt Ethan sowie Propan/Propylen befinden, welche sich zusätzlich im Waschmittel lösen.

Damit werden eine Strippung bzw. Entgasung dieser Komponenten sowie eine Rückführung dieser Komponenten zusätzlich notwendig. Dieses wiederum bedeutet erhöhte Investitions- und Betriebskosten.

Es ist daher Aufgabe der vorliegenden Erfindung, ein Verfahren der eingangs genannten Art zur Verfügung zu stellen, mit dem auf einfachere und kostengünstigere Weise Acetylen sowie Ethylen mit jeweils hohen Reinheiten hergestellt werden können.

Diese Aufgabe wird dadurch gelöst, daß im oberen Teil der Waschsäule im Rücklauf enthaltenes kondensiertes Lösungsmittel abgezogen und im unteren Drittel der Waschsäule oberhalb einer Einspeisestelle für den C₂-Strom wieder zugeführt wird.

Durch den kondensiertes Lösungsmittel enthaltenden Strom wird vom oberen Teil der Waschsäule in das untere Drittel der Waschsäule, den sogenannten "bleed-stream", eine Aufkonzentration des Waschmittels im oberen Teil der Waschsäule verhindert, wodurch eine lösungsmittelfreie Herstellung des Ethylens gewährleistet ist.

Als organische Lösungsmittel kommen alle in Frage, die ein selektives Lösungsvermögen für C₂H₂ gegenüber C₂H₄ besitzen, wobei erfindungsgemäß die Verwendung von Dimethylformanid (DMF), Aceton oder N-Methyl-Pyrrolidon (NMP) besonders günstig ist.

Gemäß einer weiteren erfindungsgemäßen Ausgestaltung des Verfahrens ist vorgesehen, daß ein Teilstrom des Rücklaufes in den oberen Teil der Waschsäule in die Waschsäule oberhalb der Einspeisestelle für den C₂-Strom und unterhalb der Einspeisestelle des Lösungsmittels eingeleitet wird.

Der Teilstrom des Rücklaufs dient auch bevorzugt zur Steuerung des Temperaturprofiles in der Waschsäule, insbesondere zur Reduzierung der Temperatur im unteren Waschabschnitt aufgrund der freiwerdenden Löslichkeitswärme.

Mit Vorteil wird das bei der Regenerierung freigesetzte Ethylen dem C₂-Strom zugemischt.

Mit besonderem Vorteil wird dabei die Regenerierung in zwei Schritten durchgeführt, wobei zuerst mittels eines Stripper-Absorbers das mitgelöste Ethylen freigesetzt, über Kopf abgezogen und zum C₂-Strom rückgeführt wird und anschließend das noch mit Acetylen beladene Lösungsmittel durch Strippen von demselben befreit und zur Wäsche rückgeführt wird.

Auf diese Weise verringert sich die zum Verdichter rückgeführte Gasmenge ganz erheblich, da lediglich reines C₂H₄ rückgeführt wird und nicht ein C₃H₆, C₃H₈, C₂H₆ und C₂H₄ enthaltender
Strom. Durch diesen verringerten Durchsatz lassen sich Energie- und Investitionskosten - z. B. bis zu 15 % bei Einsatz von Gasgemischen aus der Naphthaspaltung und bis zu 30 % bei Einsatz von Gasgemischen aus der Ethanspaltung - einsparen.

Ein weiterer Vorteil gegenüber dem bekannten Verfahren besteht darin, daß ein verbessertes Betriebsverhalten der C₂H₂-Gewinnung erreicht wird, da Störungen durch Propylen nicht mehr auftreten, weil das C₂H₂ aus einem C₃-freien Strom ausgewaschen wird.

Um Ethylen größtmöglicher Reinheit mit Bezug auf Acetylen und organisches Lösungsmittel vom Kopf der Waschsäule abziehen zu können, wird die Wäsche in vorteilhafter Weise bei Drücken von 4 bis 35 bar, vorzugsweise von 9 bis 16 bar, und entsprechenden Tautemperaturen durchgeführt.

Die Erfindung betrifft überdies eine Vorrichtung zur Durchführung des Verfahrens mit einem Verdichter 2, einer Rektifikationssäule 4, einer Waschsäule 8, einem Entspannungsbehälter 19, Wärmetauschern 18, 29, einer Stripper-Absorber-Säule 22, einem Stripper 30, Aufkochern 26, 31, Kühlern 13, 23, 37, 33, 34 sowie entsprechenden die einzelnen Vorrichtungsteile miteinander verbindenden Leitungen. In kennzeichnender Weise ist dabei die Waschsäule 8 durch einen Kaminboden 10 in zwei Teile unterteilt und eine Leitung 11 führt vom oberen in den unteren Teil der Waschsäule 8. Dadurch kann der untere Waschsäulenabschnitt so ausgelegt werden, daß die Acetylenspezifikation bezogen auf Ethylen erzielt wird, während gleichzeitig der obere Waschsäulenabschnitt so ausgelegt wird, daß durch Rückwaschung mit flüssigem Ethylen lediglich der Gehalt an organischem Lösungsmittel bezogen auf Ethylen spezifikationsgemäß eingestellt wird. Somit wird eine Reinigung des Ethylenprodukts vom Acetylen unterhalb der Nachweisgrenze (ca. 30 ppb) und vom organischem Lösungsmittel unterhalb der Nachweisgrenze (ca. 1 ppm) erreicht, was im Hinblick auf die Weiterverarbeitung von Bedeutung ist, da Acetylen und z.B. DMF Katalysatorgifte darstellen.

Nach einer weiteren Ausgestaltung der erfindungsgemäßen Vorrichtung wird in die Leitung 25 für regeneriertes Lösungsmittel, welches dem Stripper-Absorber 22 aufgegeben wird, ein zusätzlicher Kühler 24 installiert, der eine Verringerung der Verluste an organischem Lösungsmittel im Rückführgas bedingt, was wiederum zu einer Senkung der Betriebskosten und gegebenenfalls der Investitionskosten führt.

Bei der erfindungsgemäßen Anordnung ist es weiterhin vorteilhaft, den Aufkocher 26 mit Quenchwasser und Zwangsumlauf zu betreiben, um einen optimal stabilen Betrieb (d. h. im wesentlichen Temperaturstabilität) des Stripper-Absorbers 22 zu ermöglichen, was sich letztendlich positiv auf die Reinheit des zu gewinnenden Acetylens auswirkt.

Mit dem erfindungsgemäßen Verfahren läßt sich Acetylen kontinuierlich mit einer Reinheit von mindestens 99,9% produzieren, was den direkten Einsatz in weiterführenden chemischen Prozessen ermöglicht.

Insgesamt weist die Erfindung gegenüber dem bekannten Stand der Technik den Vorteil auf, daß mit optimal niedrigen Betriebs-, insbesondere Energie- und Investitionskosten innerhalb desselben Prozesses hochreines Ethylen (in Bezug auf Acetylen) und Acetylen gewonnen werden, die beide kontinuierlich direkt weiterverarbeitet werden können.

Nachstehend sei die Erfindung anhand eines schematisch dargestellten Ausführungsbeispiels näher beschrieben.

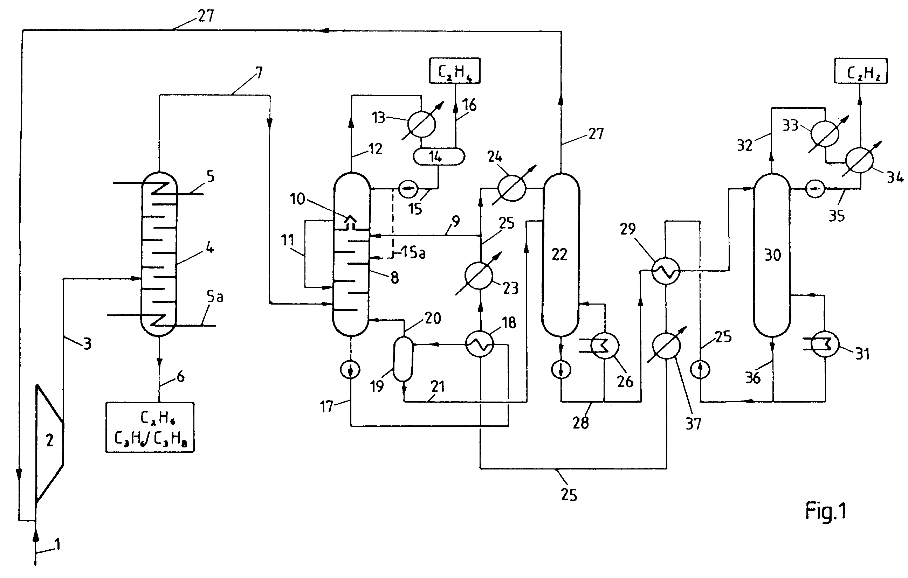

Gemäß Fig. 1 wird über Leitung 1 ein C₂-Strom, der 68 % Ethylen, 30 % Ethan, 1,9 % Acetylen und 0,1 % Propan/Propylen enthält, einem Verdichter 2 bei 8 bar zugeführt. Über Leitung 3 wird der C₂-Strom mit einem Druck von 16 bar aus dem Verdichter 2 abgezogen und einer Rektifiziersäule 4 im unteren Drittel der Säule aufgegeben. Zur Erzeugung der notwendigen Flüssigkeit sowie des notwendigen Dampfes für die Rektifikation ist die Säule 4 am Kopf und Sumpf mit Kühl- bzw. Heizschlangen 5 ausgestattet. Die Säule 4 verläßt eine Sumpffraktion (Leitung 6), die aus dem Ethan und Propan/Propylen besteht. Die zugehörige Kopffraktion wird mittels Leitung 7 abgezogen und besteht aus 97,3 % Ethylen und 2,7 % Acetylen und wird anschließend einer Waschsäule 8 zugeführt, in der das Acetylen mit Hilfe von z. B. Dimethylformamid aus dem Ethylen-Strom ausgewaschen wird. Das DMF wird über Leitung 9 zugeführt und nimmt das Acetylen im Gegenstrom zum aufsteigenden C₂-Strom auf. Des weiteren ist die Waschsäule 8 mit einem Kaminboden 10 versehen, der die Waschsäule 8 in zwei Abschnitte teilt, wobei oberhalb des Kaminbodens 10 ein Lösungsmittel enthaltender Strom 11 - der sogenannte "bleed-stream" - abgeführt und oberhalb der Einspeisestelle des C₂-Stroms (Leitung 7) der Waschsäule 8 wieder zugeführt wird.

Vom Kopf der Waschsäule 8 wird hochreines Ethylen abgezogen (Leitung 12), das nach Kühlen 13 und teilweiser Kondensation 14 zum Teil als Rücklauf auf die Waschsäule 8 aufgegeben wird (Leitung 15) und zum anderen Teil einer - nicht dargestellten - Weiterverarbeitung zugeleitet (Leitung 16) wird.

Weiterhin besteht die Möglichkeit über die Abzweigung (gestrichelt dargestellte Leitung 15a) vom flüssigen Rücklauf 15 in den unteren Waschabschnitt das Temperaturprofil in der Waschsäule zusätzlich vorteilhaft zu steuern.

Via Leitung 17 wird vom Sumpf der Waschsäule 8 mit Acetylen und mitgelöstem Ethylen beladenes DMF abgezogen, im Wärmetausch 18 zu regeneriertem Lösungsmittel angewärmt, mittels Entspannungsbehälter 19 expandiert, wobei ein Teil des mitgelösten C₂H₄ ausgast. Dieses wird in Form einer zusätzlichen thermodynamischen Trennstufe in die Waschsäule 8 rückgeführt (Leitung 20).

Das entspannte beladene Lösungsmittel (Leitung 21) wird einem Stripper-Absorber 22 aufgegeben, während oberhalb der Einspeisestelle für das beladene Lösungsmittel regeneriertes unterkühltes (Kühler 23, 24) Lösumgsmittel via Leitung 25 zugeführt wird. Gleichzeitig wird im Sumpf des Stripper-Absorbers 22 mittels des Aufkochers 26, welcher mit Quenchwasser und Zwangsumlauf betrieben wird, der nötige Strippdampf erzeugt. Auf diese Weise wird das beladene DMF vom C₂H₄ befreit, welches über Kopf abgezogen (Leitung 27) und zum Verdichter 2 rückgeführt wird.

Aus dem Sumpf des Stripper-Absorbers 22 wird nunmehr nur noch mit C₂H₂ beladenes DMF abgezogen (Leitung 28), das nach Wärmetausch 29 gegen regeneriertes Lösungsmittel der Regeneriersäule 30 oben eingespeist wird. Die Freisetzung des C₂H₂ erfolgt durch Aufkochen des Lösungsmittels (Aufkocher 31). Anschließend wird das Kopfgas der Regeneriersäule 30 über Leitung 32 mittels der Kondensatoren 33, 34 von DMF befreit. Das Kondensat wird als Rücklauf 35 der Regeneriersäule 30 am Kopf aufgegeben.

Vom Sumpf der Regeneriersäule 30 wird das vollständig regenerierte Lösungsmittel mittels Leitung 36, welche in Leitung 25 mündet, abgezogen.

Zusätzlich zu den schon beschriebenen Kühlstufen für das regenerierte Lösungsmittel 24, 23, 18, 29 ist noch ein Kühler 37 in Leitung 25 integriert, welcher die im Verfahren nicht rückgewinnbare Wärmeenergie abführt.

## Patentansprüche

1. Verfahren zur Aufarbeitung eines C₂-Stromes, der neben Ethylen als Hauptkomponente noch 15 - 30 % Ethan, 1 - 3 % Acetylen und Spuren von Propan/Propylen enthält, mittels Rektifikation bei Drücken von 4 bis 35 bar, wobei eine Kopffraktion, bestehend aus Ethylen und Acetylen, sowie eine Sumpffraktion, bestehend aus Ethan und Propan/Propylen, gewonnen wird, wobei die Kopffraktion anschließend zur Abtrennung des Acetylens einer Wäsche mit einem organischen Lösungsmittel unterworfen wird, welches nach Aufnahme des Acetylens sowie teilweise mitgelöstem Ethylens einer Regenerierung unter Freisetzung der gelösten Gase unterzogen und zur Wäsche rückgeführt wird und wobei das Kopfprodukt der Waschsäule nach Kühlung und teilweiser Kondensation zum Teil als Rücklauf in den oberen Teil der Waschsäule aufgegeben wird, **dadurch gekennzeichnet**, daß im oberen Teil der Waschsäule im Rücklauf enthaltenes kondensiertes Lösungsmittel abgezogen und im unteren Drittel der Waschsäule oberhalb einer Einspeisestelle für den C₂-Strom wieder zugeführt wird.

2. Verfahren nach Anspruch 1, dadurch gekennzeichnet, daß als organisches Lösungsmittel Dimethylformanid (DMF), Aceton oder N-Methyl-Pyrrolidon (NMP) verwendet wird.

3. Verfahren nach Anspruch 1 oder 2, dadurch gekennzeichnet, daß ein Teilstrom des Rücklaufes in den oberen Teil der Waschsäule in die Waschsäule oberhalb der Einspeisestelle für den C₂-Strom und unterhalb der Einspeisestelle des Lösungsmittels eingeleitet wird.

4. Verfahren nach einem der Ansprüche 1 bis 3, dadurch gekennzeichnet, daß der Teilstrom des Rücklaufs auch zur Steuerung des Temperaturprofiles in der Waschsäule verwendet wird.

5. Verfahren nach einem der Ansprüche 1 bis 4, dadurch gekennzeichnet, daß das bei der Regenerierung freigesetzte Ethylen dem C₂-Strom zugemischt wird.

6. Verfahren nach einem der Ansprüche 1 bis 5, dadurch gekennzeichnet, daß die Wäsche bei Drücken von 4 bis 35 bar, vorzugsweise von 9 bis 16 bar, und entsprechenden Tautemperaturen durchgeführt wird.

7. Verfahren nach einem der Ansprüche 1 bis 6, dadurch gekennzeichnet, daß die Regenerierung in zwei Schritten durchgeführt wird, wobei zuerst mittels eines Stripper-Absorbers das mitgelöste Ethylen freigesetzt, über Kopf abgezogen und zum C₂-Strom rückgeführt wird und anschließend das noch mit Acetylen beladene Lösungsmittel durch Strippen von demselben befreit und zur Wäsche rückgeführt wird.

8. Vorrichtung zur Durchführung des Verfahrens nach Anspruch 1 mit einem Verdichter (2), einer Rektifikationssäule (4), einer Waschsäule (8), einem Entspannungsbehälter (19), Wärmetauschern (18, 29), einer Stripper-Absorber- Säule (22), einem Stripper (30), Aufkochern (26, 31); Kühlern (13, 23, 37, 33, 34) sowie entsprechenden die einzelnen Vorrichtungsteile miteinander verbindenden Leitungen, dadurch gekennzeichnet, daß die Waschsäule (8) durch einen Kaminboden (10) in zwei Teile unterteilt ist und eine Leitung (11) vom oberen in den unteren Teil der Waschsäule (8) führt.

9. Vorrichtung nach Anspruch 8, dadurch gekennzeichnet, daß von einer zum Kopf der Waschsäule führenden Leitung (15) für den Rücklauf des Kondensates eine Leitung (15a) für einen Teilstrom des Rücklaufes abzweigt und in den Abschnitt der Waschsäule unterhalb des Kaminbodens (10) an einer Stelle unterhalb der Einleitung (9) des regenerierten Lösungsmittels und oberhalb der Einspeiseleitung des (7) des C₂-Stromes eingeleitet wird.

10. Vorrichtung nach Anspruch 8 oder 9, dadurch gekennzeichnet, daß in die Leitung (25) für regeneriertes Lösungsmittel, welches dem Stripper-Absorber (22) aufgegeben wird, ein zusätzlicher Kühler (24) installiert ist.

## Claims

1. A method of processing a C₂- stream, which in addition to ethylene as main component also contains 15 - 30% ethane, 1 - 3 % acetylene and traces of propane/propylene, by rectification at pressures of 4 to 35 bar, whereby a head fraction consisting of ethylene and acetylene and a bottom fraction consisting of ethane and propane/propylene is obtained, wherein, for the separation of the acetylene, the head fraction is then subjected to scrubbing with an organic solvent which, following the absorption of the acetylene and of the in part additionally dissolved ethylene, is subjected to regeneration whereby the dissolved gases are released, and is returned to the scrubbing stage, and wherein the head product of the scrubbing column, having cooled and partially condensed, is in part fed into the upper part of the scrubbing column as reflux stream, characterized in that condensed solvent contained in the reflux stream is discharged in the upper part of the scrubbing column and is returned to the lower third of the scrubbing column above a feed-in point for the C₂- stream.

2. A method as claimed in Claim 1, characterised in that dimethyl formamide (DMF), acetone or N-methyl-pyrrolidone (NMP) is used as organic solvent.

3. A method as claimed in Claim 1 or 2, characterised in that a sub-stream of the reflux into the upper part of the scrubbing column is introduced into the scrubbing column above the feed-in point for the C₂- stream and below the feed-in point for the solvent.

4. A method as claimed in one of Claims 1 to 3, characterised in that the reflux sub-stream is also used to control the temperature profile in the scrubbing column.

5. A method as claimed in one of Claims 1 to 4, characterised in that the ethylene released by the regeneration is admixed with the C₂- stream.

6. A method as claimed in one of Claims 1 to 5, characterised in that the scrubbing is carried out at pressures of 4 to 35 bar, preferably 9 to 16 bar, and corresponding dew temperatures.

7. A method as claimed in one of Claims 1 to 6, characterised in that the regeneration is carried out in two steps, wherein firstly the additionally dissolved ethylene is released by means of a stripper-absorber, is discharged at the head, and is returned to the C₂- stream, whereupon the solvent still charged with acetylene is freed of the acetylene by stripping and is returned to the scrubbing stage.

8. A device for the execution of the method claimed in Claim 1, comprising a compressor (2), a rectification column (4), a scrubbing column (8), an expansion container (19), heat exchangers (18, 29), a stripper-absorber column (22), a stripper (30), boilers (26, 31), coolers (13, 23, 37, 33, 34) and corresponding lines which connect the individual device parts to one another, characterised in that the scrubbing column (8) is divided into two sections by a chimney plate (10), and a line (11) leads from the upper into the lower part of the scrubbing column (8).

9. A device as claimed in Claim 8, characterised in that from a line (15) for the condensate reflux leading to the head of the scrubbing column, a line (15a) for a sub-stream of the reflux branches off and is introduced into the part of the scrubbing column below the chimney plate (10) at a location below the inlet line (9) for the regenerated solvent and above the inlet line (7) for the C₂- stream.

10. A device as claimed in Claim 8 or 9, characterised in that an additional cooler (24) is installed in the line (25) for regenerated solvent which is fed to the stripper-absorber (22).

## Revendications

1. Procédé pour le traitement d'un courant de C₂ qui outre de l'éthylène qui en forme le composant principal, contient également 15 à 30% d'éthane, 1 à 3% d'acétylène et des traces de propane/propylène, ce traitement consistent en une rectification sous des pressions de 4 à 35 bars pour obtenir une fraction de tête contenant l'éthylène et l'acétylène ainsi qu'une fraction de cuve contenant l'éthane et le propane/propylène, la fraction de tête étant ensuite soumise pour la séparation de l'acétylène à une étape de lavage au moyen d'un solvant organique, lequel solvant après entraînement de l'acétylène ainsi que de l'éthylène qui est partiellement dissous est soumis à une régénération en vue de libérer le gaz dissous puis est ramené à l'étape de lavage, tandis que le produit de tête de la colonne de lavage, après refroidissement et condensation partielle est en partie ramené comme reflux à la partie supérieure de la colonne de lavage, caractérisé en ce que, de la partie supérieure de la colonne de lavage, est soutirée la phase dense du solvant condensé pour comme reflux être retournée dans le tiers inférieur de la colonne de lavage au dessus du niveau auquel le courant de C₂ est amené.

2. Procédé selon la revendication 1, caractérisé en ce qu'on utilise comme solvant organique de la diméthylformamide (DMF), de l'acétone ou de N-méthylpyrrolidone (NMP).

3. Procédé selon la revendication 1 ou 2, caractérisé en ce qu'une partie du courant dense de la partie supérieure de la colonne de lavage est amenée dans la colonne de lavage au dessus du niveau d'amenée du courant de C₂ et en dessous du niveau d'amenée du solvant.

4. Procédé selon l'une des revendications 1 à 3, caractérisé en ce qu'une partie du courant dense est également utilisé pour commander le profit de température de la colonne de lavage.

5. Procédé selon l'une des revendications 1 à 4, caractérisé en ce que l'éthylène libéré dans la régénération est mélangé au courant de C₂.

6. Procédé selon l'une des revendications 1 à 5, caractérisé en ce que le lavage est effectué sous des pressions de 4 à 35 bars, de préférence de 9 à 16 bars, aux températures de rosée correspondantes.

7. Procédé selon l'une des revendications 1 à 6, caractérisé en ce que la régenération est effectuée en deux étapes, l'éthylène entraîné à l' état dissous par le solvant étant tout d'abord libéré au moyen d'un strippeur-absorbeur, soutiré en tête et réuni au courant de C₂ et ensuite le solvant encore chargé d'acétylène en est libéré par strippage puis est recyclé vers la zone de lavage.

8. Dispositif pour la mise en oeuvre du procédé selon la revendication 1, comprenant un condenseur (2), une colonne de rectification (4), une colonne de lavage (8), un vase de détente (19), des échangeurs thermiques (18, 29), une colonne de strippage absorption (22), un strippeur (30), des rebouilleurs (26, 31), des refroidisseurs (13, 23, 37, 33, 34), ainsi des canalisations correspondantes reliant entre elles différentes parties du dispositif, caractérisé en ce que la colonne de lavage (8) est divisée en deux parties par une section en forme de cheminée (10) et en ce qu'une canalisation (11) relie de la partie supérieure et la partie inférieure (8) de la colonne.

9. Dispositif selon la revendication 8, caractérisé en ce que d'une canalisation (15) conduisant en tête de colonne de lavage, comme reflux de condensat, dérive une canalisation (15a) pour une partie du courant de reflux qui est amenée à la section de la colonne de lavage se trouvant située au-dessous de la section en forme de cheminée (10) dans laquelle il pénètre à un niveau situé au-dessous de la conduite d'amenée du solvant régénéré et situé au dessus de la conduite d'amenée (7) du courant de C₂.

10. Dispositif selon les revendications 8 ou 9, caractérisé en ce que dans la canalisation (25) du solvant régénére, lequel est conduit au strippeur-absorbeur (22), est disposé un refroidisseur supplémentaire (24).
